# EUROPEAN PATENT APPLICATION

(11) **EP 0 919 191 A2**
(43) Date of publication of application: **02.06.1999**
(21) Application number: 98309547.2
(22) Date of filing: 23.11.1998
(51) Int. Cl.: A61B 10/00

(54) **Biopsy assembly including an expandable sleeve**

(30) Priority: 24.11.1997 US 976817
(71) Applicant: ETHICON ENDO-SURGERY, Cincinnati, Ohio 45242-2839 (US)
(72) Inventor: Roresster, Graig F., Mason, Ohio 45040 (US)
(74) Representative: Mercer, Christopher Paul

(57) **Abstract**

A surgical instrument assembly for excising a targeted tissue mass to be biopsied from adjacent bodily tissue of a surgical patient is disclosed. The assembly has a tissue biopsy device and an expandable sleeve (11) for receiving the tissue biopsy device. The biopsy device has a penetrating member affixed to a distal end of an elongated tube to facilitate insertion of the tube adjacent the targeted tissue mass. It also has a tissue receiving chamber at the distal end of the tube for receiving the mass and a tissue cutter movable relative to the penetrating member from first to second positions for excising the targeted tissue mass. The expandable sleeve provides an access portal to the targeted tissue mass. It is expandable from a first original diameter to a second expanded diameter in response to insertion of the tissue biopsy device where at least a portion of the device has a device diameter corresponding to the second expanded diameter. When the tissue biopsy device is inserted through the sleeve, and the sleeve expands, the surrounding tissue is dilated. The dilation of tissue is less traumatic to the patient than incisional length which matches the diameter of the penetrating member of the biopsy device.

## Description

### Background of Invention

This invention relates to an assembly for facilitating the removal of a suspect lesion for a subsequent biopsy to determine malignancy. More particularly, it relates to such an assembly which can excise the suspect lesion and remove it for subsequent biopsy in a minimally invasive manner.

Statistics currently reveal that one in nine American women will develop breast cancer. It is the leading cause of cancer deaths in women between the ages of 40-55, and the second leading cause of cancer deaths in women overall. Unfortunately, breast cancer will only be diagnosed in about one in eight women, and one in thirty will die of this disease. Breast cancer does occur in men but it is much more uncommon.

A biopsy of the breast is often indicated if suspicious tissue is detected. Biopsy requests stem from a screening process generally performed via a physical examination to detect a palpable lesion or a mammogram for the detection of a nonpalpable lesion. Five out of six biopsies performed return benign indications.

The goal of the biopsy is the discovery and accurate diagnosis of benignity or malignancy of suspect tissue. Survival rates are significantly greater with early detection. However, as detection is attempted in earlier stages of carcinoma development, accurate sampling of the lesion becomes more difficult due to small lesion size. Ideally, a sufficiently large sample size is taken to accurately diagnosis disease state with minimal trauma (physical deformity or scarring) to the patient.

The current standard for care is a significantly invasive procedure known as "Open Excisional Biopsy". Prior to an open excisional biopsy for a nonpalpable mass, an interventional radiologist will initially take mammograms to place a guidewire which directs the surgeon to the area of excision. Mammograms are then taken to assure that the guidewire is accurately placed in or near the desired biopsy site. The guidewire has a barbed end in order maintain its position at the desired site and is fed through an elongated needle.

The general surgeon applies local or, more commonly, general anesthesia, during an open excisional biopsy. Dissection is made through the skin and subcutaneous fat and the flap is undercut and retracted. Incision length, during an open excisional biopsy generally runs between 2-5 inches and is dependent on the location of the lesion, breast characteristics, and surgeon technique. The breast parenchyma is then dissected along the guidewire until the site of the specimen is exposed. The specimen, generally spherical in shape centered about the barbed end of the guidewire, and roughly 1 inch in diameter, is manually excised.

The specimen size taken during an open excisional biopsy is adequate, however deformation (scanning and subsequent shape of the breast) and potential "seeding" of the cancerous cells is often a large concern. Accuracy is generally acceptable, however the guidewire often moves from its original position during the excisional process leading to decreased accuracy and the need for larger specimens.

New technologies are being developed to assist the early detection of breast cancer. The technologies are primarily focused on imaging of nonpalpable tissue and accessing the tissue with minimally invasive techniques for biopsy. The cost and trauma associated with open excisional biopsy is minimized with these technologies.

The two primary imaging technologies for assisting minimally invasive breast biopsy of a nonpalpable mass are ultrasound and stereotactic X-rays. Ultrasound accurately guides a hand-held core biopsy needle (CBN) to the biopsy site. Stereotactic imaging is performed with the patient lying prone on a stereotactic table and X-rays are teen at 15 degrees off-axis on each side of the breast. The X-rays are fed into a digital signal imaging system to create a 3-D reference; the location for biopsy is triangulated and coordinates are fed to the stereotactic table.

The actual specimen removal in the minimally invasive approach is performed in any one of the following ways.

TruCut® Needle Method - The "TruCut" needle method utilizes a small diameter needle which is inserted to a desired depth, actuated, and removed. The product for practicing this method has two co-axial tubular members. The outer member is retracted exposing a lateral opening in the inner member. The elastic property of the tissue causes tissue to enter the hollow core of the inner member. The outer member is then advanced forward, shearing the tissue sample which has been trapped in the inner member. Inherent disadvantages to this technique are the need for multiple insertions with questionable accuracy and extremely limited tissue sample sizes. The TruCut® needle and its method of use are described in U.S. Patent 4,958,625.

Surface to Site Core Sampling (SSCS) - The SSCS method attempts to maximize accuracy through the increase in sample size while maintaining controlled guidance. A cylindrical cutting tube is advanced through an opening in the skin. As it moves forward, it creates a continuous cylindrical core sample until it reaches the depth desired by the surgeon. Upon completion of the linear motion, the distal end of the sample is transected, and the device is withdrawn with the sample within the cutting element. The critical disadvantage to this approach is the trauma and disfigurement associated with excision of the healthy tissue superior to the site of questionable tissue.

Percutaneous Core Biopsy (PCB) - The PCB method attempts to obtain the advantages of single insertion and large sample size of the SSCS approach, while minimizing disfigurement as achieved by the TruCut® needle method. As in the case of a TruCut® needle device, the PCB has multiple co-axial tubular members. However, one of the members remains stationary, eliminating the need for multiple insertions. Samples are obtained in a controlled contiguous manner which ultimately achieves the desire for obtaining large tissue samples at the questionable site.

A recent minimally invasive approach for excising a portion of a targeted lesion is described in U.S. Patent 5,526,822. The biopsy apparatus described in this patent has an outer biopsy cannula with a penetrating member for positioning adjacent the targeted lesion. The biopsy cannula has a lateral tissue receiving port, and a vacuum is drawn through the port for pulling tissue into the port. An inner cutting cannula moves forwardly to excise the tissue received in the port. With the aid of a vacuum drawn on the inner cutting cannula, the inner cutting cannula retains the excised tissue as the inner cannula is retracted for tissue removal, and the outer biopsy cannula remains stationary. Following removal, the outer biopsy cannula can be rotated relative to a fixed housing, and multiple samples can therefore be taken circumferentially without the need for multiple insertions and withdrawals of the biopsy cannula. The biopsy apparatus and its method of use as described in the '822 patent are exemplified in the Mammotome® Breast Biopsy System.

Although minimally invasive approaches for obtaining biopsy samples are becoming more wide spread, there is still a need for an instrument assembly which will further reduce the trauma associated with biopsies and yet still capture sufficiently sized biopsy samples. In particular, what is needed is an instrument assembly which can further reduce the incision, penetration and trauma associated with the insertion of the biopsy instrument through the tissue layers for positioning adjacent the biopsy site for the capture of the intended or targeted lesion. In particular, such an instrument assembly would be especially desired if it could reduce this trauma without sacrificing sample size.

### Summary of the Invention

The invention is a surgical instrument assembly for excising a targeted tissue mass to be biopsied from adjacent bodily tissue on a surgical patient. The assembly comprises a tissue biopsy device and an expandable sleeve.

The tissue biopsy device has an elongated tube, a penetrating member, a tissue receiving chamber and a tissue cutter. The elongated tube has proximal and distal ends. When the instrument is positioned to excise the targeted tissue mass, the proximal end of the elongated tube is positioned externally of the surgical patient and the distal end of the elongated tube is positioned adjacent the targeted tissue mass. The penetrating member is affixed to the distal end of the elongated tube. The penetrating member has a penetrating tip at a distal end of the penetrating member to facilitate the insertion of the distal end of the elongated tube adjacent the targeted tissue mass. The tissue receiving chamber is in communication with the distal end of the elongated tube for receiving the targeted tissue mass to be biopsied or a portion thereof. The tissue cutter has a distal cutting edge surface. The cutter is movable relative to the penetrating member from a first position where the cutting edge surface is disposed proximally of the tissue receiving chamber to a second position located distally of the first position where the cutting edge surface has excised the targeted tissue mass to biopsied or the portion thereof within the tissue chamber from the adjacent bodily tissue.

The expandable sleeve receives the tissue biopsy device for providing an access portal to the targeted tissue mass. The sleeve is expandable from a first original diameter to a second expanded diameter in response to insertion of the tissue biopsy device with at least a portion thereof having a device diameter corresponding to the second expanded diameter.

The expandable sleeve of the surgical instrument assembly of this invention for excising the targeted tissue mass to be biopsied may significantly reduce the trauma associated with the insertion and withdrawal of the tissue biopsy device for capturing and removing the targeted tissue mass from the biopsy site. It reduces the trauma because unlike conventional tissue biopsy devices where the incisional length needed for insertion of the penetrating member of the tissue biopsy device must match the diameter of the penetrating member, the expandable sleeve of the assembly of this invention enables a smaller incision in combination with the dilation of tissue for the insertion and withdrawal of the tissue biopsy device. The dilation of the tissue, in contrast to enlarge incision, causes significantly less trauma to the surgical patient. Further, this reduction in patient trauma can be realized without sacrificing the desire for a large specimen size because the tissue receiving chamber of the biopsy device does not need to be designed with less tissue receiving space to correspond with the size of the incision since the assembly relies on tissue dilation through the expandable sleeve acting as a portal.

The surgical instrument assembly of this invention can be used during any surgical procedure where it is necessary or desirable to excise a targeted tissue mass for biopsy from adjacent bodily tissue. In particular, the assembly is especially well suited for minimally invasive applications where it is desirable to obtain a biopsy while significantly minimizing the trauma to the patient.

### Brief Description of the Drawings

Figure 1 is an isometric view of the distal end of a preferred embodiment of a tissue biopsy device which can be used in the instrument assembly of this invention.
Figure 2 is a centerline section view of the preferred tissue biopsy device prior to insertion into the expandable sleeve of the instrument assembly of this invention. The expandable sleeve is illustrated inserted into the breast tissue of a patient and dircted to a tissue mass subject to biopsy.
Figure 3 is a centerline section view of the assembly where the device is inserted into the sleeve.
Figure 4 is a centerline section view of the device fully inserted into the expandable sleeve. The distal end of the sleeve is adjacent the targeted tissue mass.

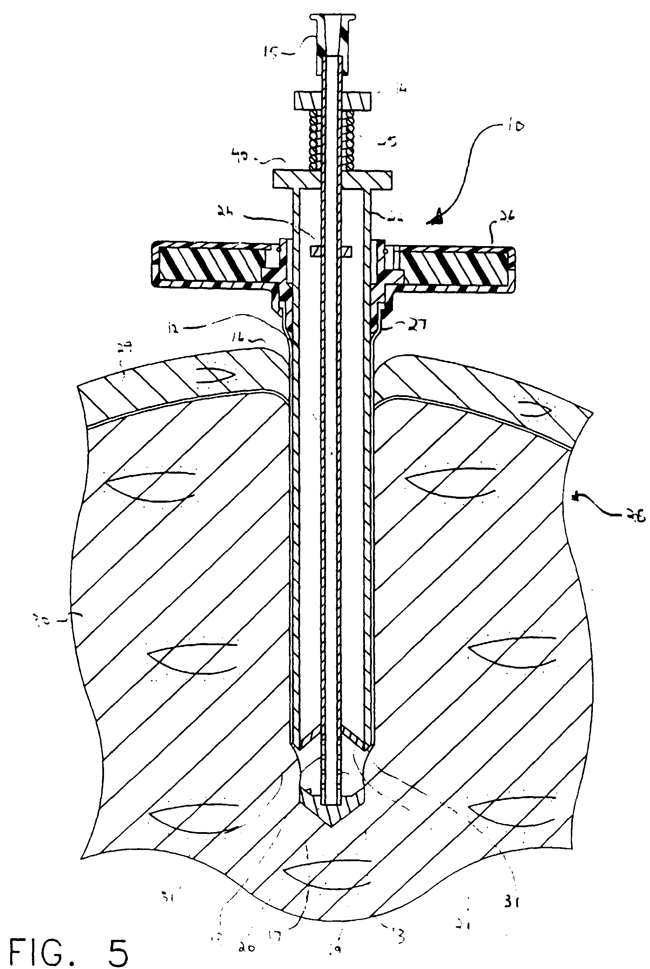

Figure 5 is a centerline section view of the assembly where the distal end of the device extends from the sleeve and into the targeted tissue mass.
Figure 6 is a centerline section view of the assembly where vacuum pressure is applied to the device through the tissue receiving chamber, thus drawing in a sample mass of tissue.
Figure 7 is a centerline section view illustrating the distal movement of the tissue cutter to excise the suspect tissue mass.

### Detailed Description of the Preferred Embodiment

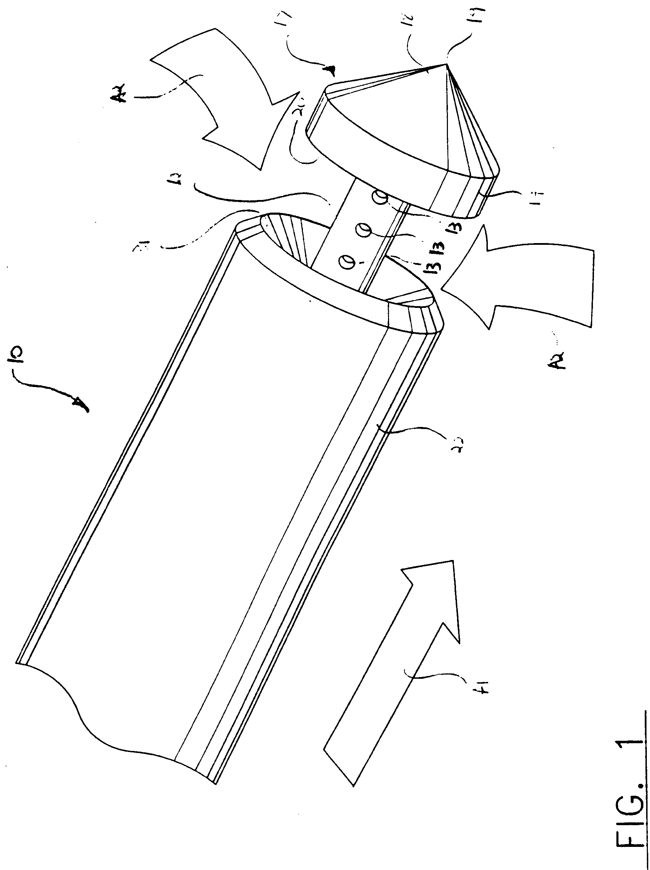

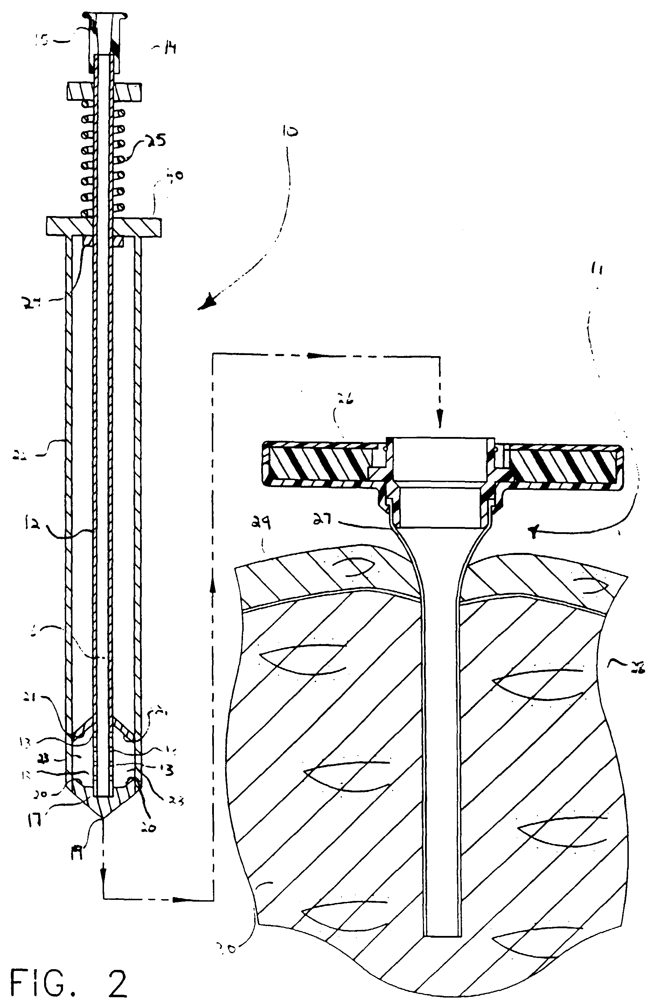

A perspective view of the preferred surgical instrument, specifically the tissue biopsy device 10 which is advantageously used with an expandable sleeve 11 to readily provide access to the targeted tissue mass, is shown in Figure 1. Figure 2 provides additional details in a sectional view of the tissue biopsy device and the expandable sleeve, as well as depicting the interrelationship between the device and the sleeve.

The biopsy device 10 has an elongated tube 12 with proximal and distal ends which extend from the proximal and distal ends of the device. At the distal end of the tube, there are a plurality of vacuum orifices 13 to pull a vacuum against the distal end of the tube. At the proximal end of the tube, there is a tube base 14 and a vacuum adapter 15 which is connected to a vacuum source for drawing the vacuum through the vacuum orifices and through the longitudinal channel 16 of the elongated tube (the vacuum source is not shown in Figures 1 or 2).

Affixed to the distal end of the tube of the surgical biopsy instrument is a penetrating member 17. The penetrating member has a sharp penetrating tip 18, and the penetrating tip has a conical configuration. The penetrating member has a circular base 19 at its proximal end. The base is spaced from the apex 19 of the penetrating tip. The underside of the base of the penetrating member acts as a first tissue stop 20. The first tissue stop extends outwardly in the radial direction from the elongated tube. A second tissue stop 21 extends from the elongated tube and faces the first tissue stop (see Figure 2). Similarly to the first tissue stop, the second tissue stop extends outwardly in the radial direction from the elongated tube, and is located proximally of the first tissue stop.

The annular tissue cutter 22 of the biopsy device is mounted concentrically, about the elongated tube. It is movable longitudinally relative to the penetrating member affixed to the tube from a first non-cutting position to a second tissue-cut position. In Figure 2, the tissue cutter is shown in its first non-cutting position. In this position, the tissue cutter is located adjacent the base of the penetrating member, and the gap between the first and second tissue stops is enclosed.

The first and second tissue stops define a tissue receiving chamber 23 between the stops. This receiving chamber can receive tissue continuously about a 360° arc at the distal end of the tube where the vacuum orifices are placed. A greater amount of tissue can be loaded into the receiving chamber than what is capable if a conventional lateral tissue receiving port were used. Further, the outward radial dimension provided by the first and second tissue stops also increases the depth of tissue which can be loaded into the chamber.

The annular cutter 22 of the device has a cutter base 40 at a proximal end and a cutter stop 24 located adjacent to the cutter base. Interposed between the tube base and the cutter base is a cutter spring 25. The spring biases the annular cutter in the distal direction. Consequently, the spring biases the cutter toward its first non-cutting position.

The sleeve 11 which is used to receive the tissue biopsy device for providing the access portal to the target tissue mass is expandable. The sleeve is seed to receive the biopsy device and has a lengthwise dimension sufficient to extend to a position where the distal end of the sleeve is adjacent the targeted tissue mass. At a proximal end of the sleeve, there is a sleeve handle 26 for gripping and manipulating the sleeve. There is an expanded base region 27 transitioning between the sleeve handle 26 and the expandable sleeve 11. The sleeve is inserted into the tissue 28 to a location adjacent the target tissue mass using conventional means. When a breast biopsy is desired, the sleeve is inserted through the outer skip layer 29 of the breast and into the percutaneous breast tissue 30. When the sleeve is fully inserted, the sleeve handle 26 is located exteriorly of the outer skin layer 29 of the breast.

Importantly, the sleeve is expandable from a first original diameter to a greater, second expanded diameter to accomodate the diameter of the annular cutter of the biopsy device. The sleeve is originally inserted into the breast at its first original diameter, and then it is expanded to its second diameter when the biopsy device is inserted through the sleeve. The expanded second diameter is substantially the same as the diameter of the annular cutter of the device.

An expandable sleeve which can be used in the practice of this invention is described in U.S. Patent Nos. 3,789,852; 4,716,901; 4,899,729; 5,183,464 and 5,320,611.

As depicted in Figure 1, the tissue biopsy device 10 is inserted in the direction depicted by directional arrow A1 to take advantage of the conical tip configuration of the penetrating member to facilitate ease of insertion. When the vacuum is pulled through the vacuum orifices against the distal end of the tube, tissue can be pulled into the tissue receiving chamber in the direction depicted by the directional arrows A2 as shown.

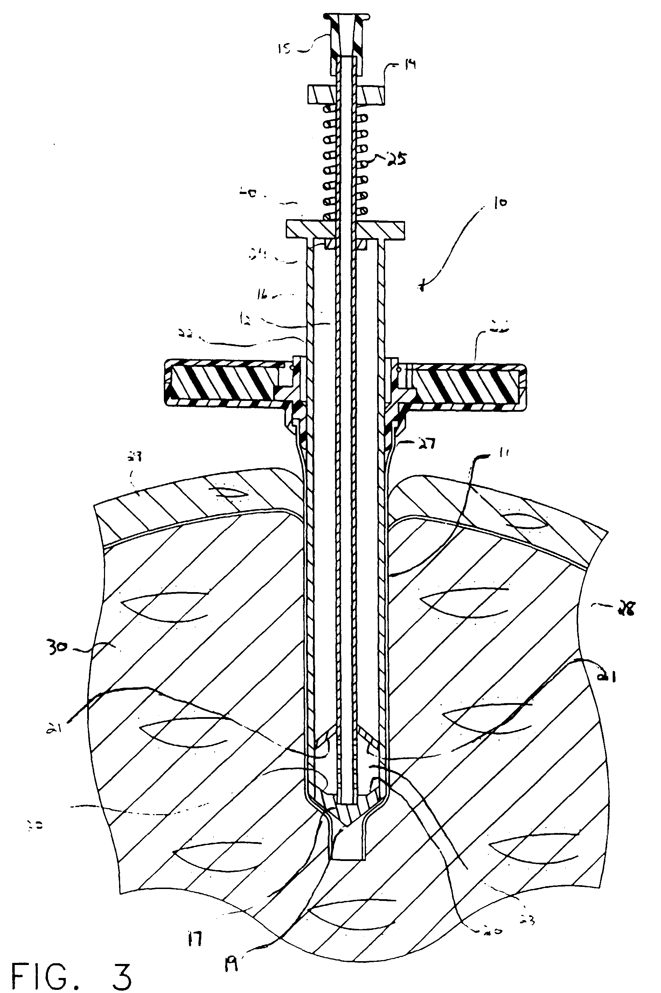

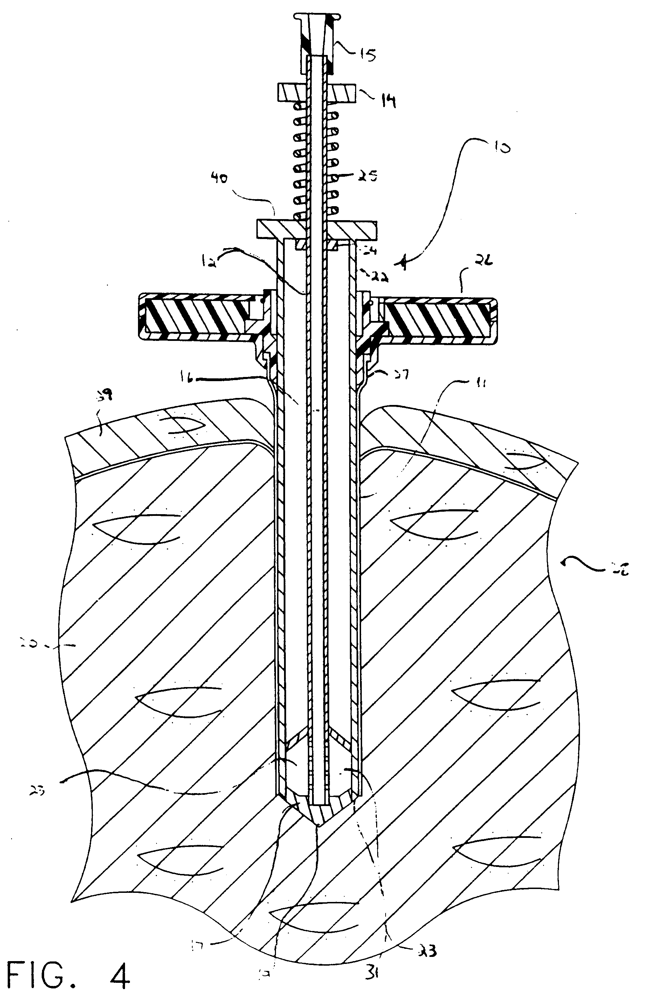

When the sleeve has been inserted into the tissue at its first original diameter, the tissue biopsy device can then be inserted through the sleeve, using the sleeve as an access portal to provide ease of insertion of the tissue biopsy device to a location where the tissue receiving chamber can be placed within the targeted tissue mass. As shown in Figure 3, when the biopsy instrument is inserted into the sleeve, the sleeve expands from its first original diameter to its second expanded diameter which corresponds to the diameter of the annular tissue cutter. In so doing, the tissue is dialated from the first original diameter to the second expanded diameter, causing less trauma to the patient than if an incision the size of the second expanded diameter were made. In Figure 4, the biopsy device is fully inserted and the penetrating tip of the penetrating member affixed to the elongated tube extends from the distal end of the expanded sleeve. Additionally, when fully inserted, the proximal end of the tube of the biopsy device is positioned externally of the tissue, and the tube distal end is positioned percutaneously adjacent the targeted tissue mass.

Once the tissue biopsy instrument is inserted fully through the expanded sleeve, the annular cutter 22 is retracted from its first non-cutting position to its second tissue-cut position. A distal cutting edge surface 31 at the distal end of the annular cutter is exposed. This surface is located adjacent the first tissue stop when the cutter is retracted to its second tissue-cut position. The cutter is retracted by exerting an upper proximal force against the spring 25 (using the cutter base 40 as a grip to apply the force) to compress the spring. The cutter is then held in its second position. In the meantime, the elongated tube 12 of the tissue biopsy device, and the expandable sleeve 11, remain stationary relative to the movement of the annular tissue cutter 22.

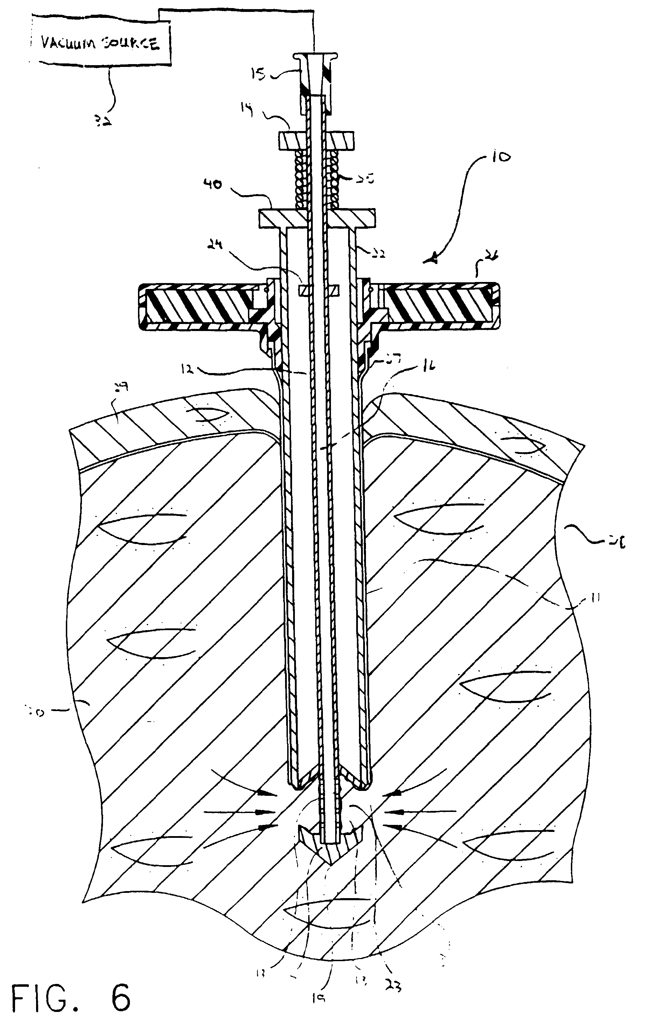

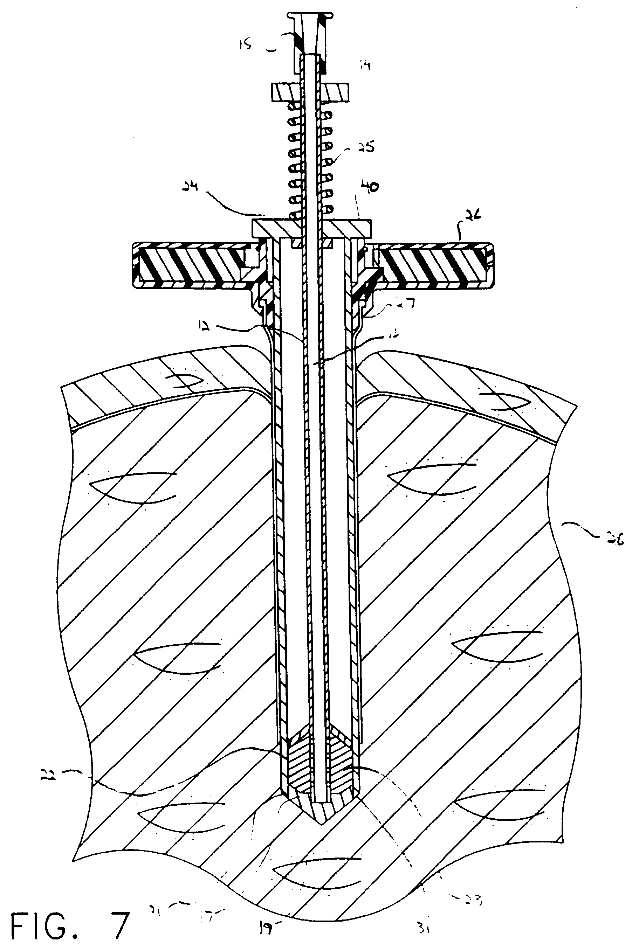

Referring now to Figure 6, with the annular cutter 22 in its retracted, second tissue-cut position, the vacuum adapter 15 is connected to a vacuum source 32 (see Figure 6), and vacuum is applied. When the vacuum is applied, at least a portion of the targeted tissue mass is pulled into the tissue receiving chamber and against the distal end of the elongated tube as illustrated by the directional arrows depicted in Figure 6. The vacuum is drawn through the vacuum orifices and into the longitudinal channel 16 of the elongated tube 12. Once the tissue is loaded into the tissue receiving chamber 23 as a consequence of the application of vacuum, the annular cutter can be released from its second position. When it is released, the distal cutting edge surface 31 of the annular cutter excises the tissue loaded into the chamber as the cutter slides longitudinally from its second tissue-cut position to its first non-cutting position. When the annular cutter returns to its first non-cutting position, the desired portion of the targeted tissue mass is fully enclosed within the continuous tissue receiving chamber of the device as depicted in Figure 7. From this point, the device can be removed from the sleeve, and the sample of tissue in the chamber can be extracted from the chamber using conventional techniques.

Although this invention has been described in connection with its most preferred embodiment, this particular embodiment is provided by way of example, and should not be used to limit the scope of the claimed invention. The scope of the invention is defined by the claims which follow.

## Claims

1. A surgical instrument assembly for excising a targeted tissue mass to be biopsied from adjacent bodily tissue of a surgical patient, said assembly comprising:
a) a tissue biopsy device having: i) an elongated tube having proximal and distal ends, and when said instrument is positioned to excise the targeted tissue mass, the proximal end of said elongated tube is positioned externally of the surgical patient and the distal end of said elongated tube is positioned adjacent the targeted tissue mass; ii) a penetrating member affixed to the distal end of said elongated tube, said penetrating member having a penetrating tip at a distal end of said penetrating member to facilitate the insertion of the distal end of said elongated tube adjacent the targeted tissue mass; iii) a tissue receiving chamber in communication with the distal end of said elongated tube for receiving the targeted tissue mass to be biopsied or a portion thereof, and iv) a tissue cutter having a distal cutting edge surface, said cutter movable relative to said penetrating member from a first position wherein said cutting edge surface is disposed proximally of said tissue receiving chamber to a second position located distally of said first position wherein said cutting edge surface has excised the targeted tissue mass to be biopsied or the portion thereof within said tissue receiving chamber from the adjacent bodily tissue; and
b) an expandable sleeve receiving said tissue biopsy device for providing an access portal to the targeted tissue mass, said sleeve being expandable from a first original diameter to a second expanded diameter in response to insertion of said tissue biopsy device with at least a portion thereof having a device diameter corresponding to said second expanded diameter.
